(19) Europäisches Patentamt
European Patent Office
Office européen des brevets

(11) Publication number: 0 652 217 A1

(12) **EUROPEAN PATENT APPLICATION**

(21) Application number: 94117034.2

(51) Int. Cl.[6]: C07D 405/12, A61K 31/415

(22) Date of filing: 27.10.94

(30) Priority: 08.11.93 JP 278215/93

(43) Date of publication of application: 10.05.95 Bulletin 95/19

(84) Designated Contracting States: AT BE CH DE DK ES FR GB GR IE IT LI LU NL PT SE

(71) Applicant: MITSUBISHI CHEMICAL CORPORATION
5-2 Marunouchi 2-chome
Chiyoda-ku,
Tokyo 100 (JP)

(72) Inventor: Takashima, Junko, c/o Mitsubishi Chem. Corporation
Yokohama Res. Center,
1000, Kamoshida-cho,
Aoba-ku
Yokohama-shi,
Kanagawa-ken (JP)
Inventor: Uchida, Shoko, c/o Mitsubishi Chem. Corporation
Yokohama Res. Center,
1000, Kamoshida-cho,
Aoba-ku
Yokohama-shi,
Kanagawa-ken (JP)

(74) Representative: TER MEER - MÜLLER - STEINMEISTER & PARTNER
Mauerkircherstrasse 45
D-81679 München (DE)

(54) **N-substituted imidazole derivatives.**

(57) The N-substituted imidazole derivatives of the following formula (1)

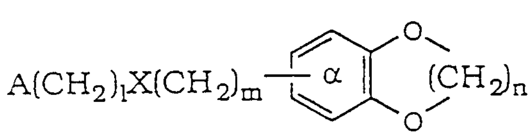

... (I)

wherein A is

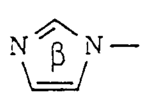 or 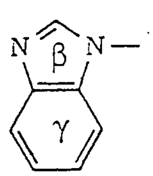 ;

X is -O-, -NHCO- or -NH-, 1 is an integer of 2 to 6; m is 0 or 1; n is 1 or 2;ring $\alpha$, ring $\beta$ and ring $\gamma$ are independently unsubstituted or substituted by one or more substituents selected from the group consisting of a halogen atom, $C_1$ ~ $C_3$ alkyl, $C_1$ ~ $C_3$ alkoxy and cyano; or its pharmaceutically acceptable salt, or their hydrates, show excellent anti-hypertensive, anti-hyperlipemic and blood sugar lowering activity, and are useful as excellent antihypertensives, preventives or remedies for ischemic cardiac diseases such as angina, myocardial

EP 0 652 217 A1

infarction, heart failure, arteriosclerosis, or as preventives or remedies for diabetes and complications thereof.

## BACKGROUND OF THE INVENTION

### 1. Field of the Invention

The present invention relates to N-substituted imidazole derivatives. More particularly, it relates to N-substituted imidazole derivatives having hypoglycemic hypolipidaemic and antihypertensive activity.

### 2. Background of the Invention

Hypertension is an important risk factor in circulatory diseases. Recently, development of various types of depressors has made it relatively easy to lower blood pressure level of hypertensive patients. Up to date, however, administration of such depressors do not reduce incidence of myocardial infarction and sudden death. Accordingly, development of depressors which reduce incidence of myocardial infarction and sudden death has been expected.

## SUMMARY OF THE INVENTION

The present inventors have studied intensively to provide novel drugs for circulatory diseases to reduce incidence of myocardial infarction or sudden death. As the results, we have found that N-substituted imidazole derivatives of the present invention have hypoglycemic, hypolipidaemic and anti-hypertensive activity, and have attained the present invention.

That is, the present invention provides N-substituted imidazole derivatives of the following general formula (I):

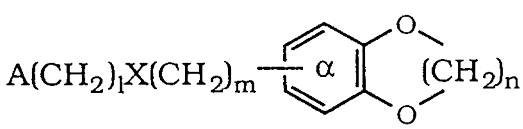

... (I)

wherein A is

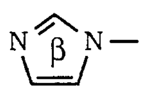 or 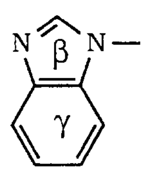 ;

X is -O-, -NHCO- or -NH-, 1 is an integer of 2 to 6; m is 0 or 1; n is 1 or 2; ring α, ring β and ring γ are independently unsubstituted or substituted by one or more substituents selected from the group consisting of a halogen atom, $C_1 \sim C_3$ alkyl, $C_1 \sim C_3$ alkoxy and cyano; or pharmaceutically acceptable salt, or hydrates thereof.

## DETAILED DESCRIPTION OF THE INVENTION

The present invention will be explained in detail.

In the compounds represented by the above general formula (I), $C_1 \sim C_3$ alkyl groups substitutable on rings α, β and γ include, for example, methyl, ethyl, n-propyl, isopropyl, preferably methyl. The position of substitution is particularly preferably at 2-position of an imidazole ring represented by ring β.

$C_1 \sim C_3$ Alkoxy groups substitutable on rings α, β and γ include, for example, methoxy, ethoxy, n-propoxy, isopropoxy, preferably methoxy. The position of substitution is preferably at 3 or 6-position of a benzene ring represented by ring α.

Halogen atoms substitutable on rings α, β and γ include, for example, fluorine, chlorine and bromine, preferably chlorine. The position of cyano group substituted on rings α, β and γ is preferably 4- and 5-positions of an imidazole ring represented by ring β.

All of ring α, ring β and ring γ are more preferably unsubstituted.

A is preferably

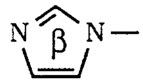 .

X is preferably -O- or -NHCO-, more preferably -O-. The preferred compounds according to the present invention are those of the above general formula (I) wherein 1 is an integer of 3 to 5, m is 0, and n is 1.

Among the compounds of the present invention, particularly preferred compounds include,
4-[3-(4,5-dicyano-1-imidazolyl)propoxy]-1,3-benzodioxole;
4-[3-(4,5-dichloro-1-imidazolyl)propoxy]-1,3-benzodioxole;
4-[3-(1-benzimidazolyl)propoxy]-1,3-benzodioxole;
4-[4-(1-imidazolyl)butoxy]-1,3-benzodioxole;
4-[5-(1-imidazolyl)pentoxy]-1,3-benzodioxole;
N-[3-(1-imidazolyl)propyl]-1,4-benzodioxane-6-carboxamide;
or salts thereof.

Salts of N-substituted imidazole derivatives of the present invention represented by the above general formula (I) are pharmaceutically acceptable salts with mineral or organic acid and include, for example, hydrochloride, sulfate, nitrate, acetate, oxalate, tartrate, citrate, lactate, p-toluenesulfonate, methanesulfonate and the like. In addition, N-substituted imidazole derivatives of the above general formula (I) or salts thereof according to the present invention may form hydrates.

The compound (I) of the present invention can be produced by the following process 1 to 3:

(Process 1)

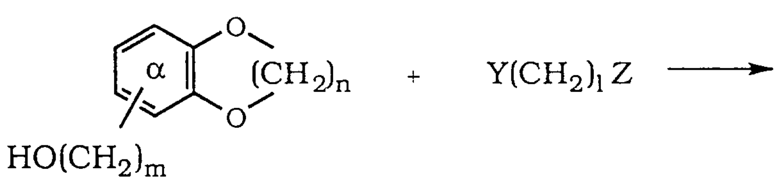

(II) (III)

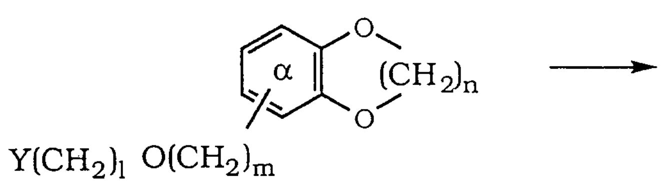

(IV)

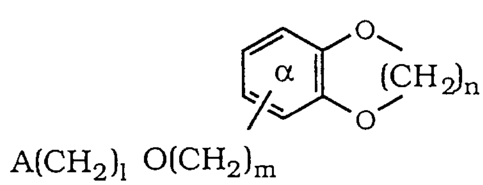

(V)

(wherein, A, α, 1, m and n are as defined in the above general formula (I); Y and Z are independently halogen atoms or ester groups such as sulfonate ester, for example, chlorine atom, bromine atom, iodine atom, methanesulfonyloxyl, p-toluenesulfonyloxy, trifluoromethanesulfonyloxy, acetoxy, etc.).

This process comprises linking straight chain alkyl having a leaving group substituted at the end to OH group of phenol derivative or benzyl alcohol derivative which may have substituents, and substituting the leaving group with imidazole or benzimidazole which may have substituents, to obtain the objective

compound (V).

The reaction to link straight chain alkyl group having a leaving group substituted at the end to OH group of phenol derivative or benzyl alcohol derivative which may have substituents in the first step may be generally carried out in organic solvent in the presence of a base.

The usable bases include, for example, sodium hydroxide, potassium hydroxide, sodium carbonate, potassium carbonate, sodium hydride, sodium alkoxide, triethylamine, etc.

The usable organic solvents include, for example, acetonitrile, tetrahydrofuran, ether, acetone, dimethyl sulfoxide, dioxane, N,N-dimethylformamide, methanol, ethanol, isopropanol, tert-butanol, etc. Alkylating agents include, for example, ω-chloroalkyl chloride, ω-chloroalkyl bromide, ω-chloroalkyl iodide, ω-chloroalkyl tosylate, ω-chloroalkyl mesylate, etc.

The reaction temperature and time may not be particularly limited. The reaction may be generally carried out at suitable temperature from ice-cooling to refluxing temperature for 15 minutes to 72 hours.

Thus obtained compound may be used in the subsequent reaction after conventional means such as extraction with solvent, separation by chromatography, crystallization, etc.

Subsequently, the resulting compound (IV), neat or as a solution in a suitable organic solvent, is generally linked to an imidazole ring or a benzimidazole ring which may have substituents generally in the presence of a base to obtain a compound (V).

The bases used in this step include, for example, sodium hydroxide, potassium hydroxide, sodium carbonate, potassium carbonate, sodium hydride, sodium alkoxide, triethylamine, etc.

The organic solvents used include, for example, acetonitrile, tetrahydrofuran, ether, acetone, dimethyl sulfoxide, dioxane, N,N-dimethylformamide, methanol, ethanol, isopropanol, etc.

When the reaction is carried out using a neat material, good results may be obtained using phase transfer catalyst. Such catalysts include, for example, aliquot 336, tetra-n-butylammonium bromide, tetra-n-octylammonium bromide, etc. The reaction temperature and time may not be particularly limited. The reaction may be carried out at suitable temperature from ice-cooling to refluxing temperature for 15 minutes to 72 hours.

Thus obtained compound may be purified by the conventional means such as extraction with solvent, separation by chromatography, crystallization, etc.

(Process 2)

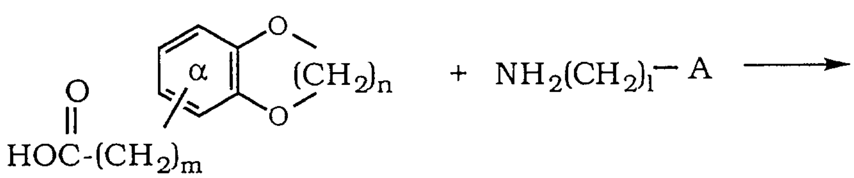

(VI) (VII)

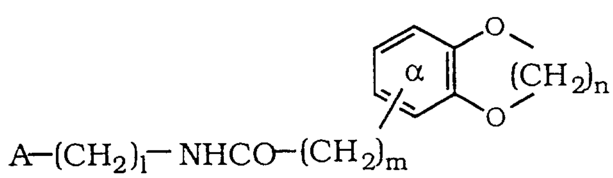

(VIII)

(wherein, A, α, 1, m and n are as defined in the above general formula (I)).

This process comprises condensation of carboxyl group of benzoic acid derivative or phenylacetic acid derivative which may have substituents with straight chain alkyl amine having an imidazole ring or a benzimidazole ring at the end which may have substituent, to obtain the objective compound (VIII).

In the reaction to synthesize amide (VIII) from carboxylic acid (VI) and amine (VII), carboxylic acid may be converted to a carboxylic acid derivative such as acid chloride, acid anhydride, ester, amide, etc., which is then reacted with amine. Alternatively, carboxylic acid (VI) may be directly subjected to dehydration with amine (VII) to give the objective amide (VIII).

Isolation and purification of thus obtained compound (VIII) may be carried out, for example, by extraction with solvent, separation by chromatography, crystallization, etc.

(Process 3)

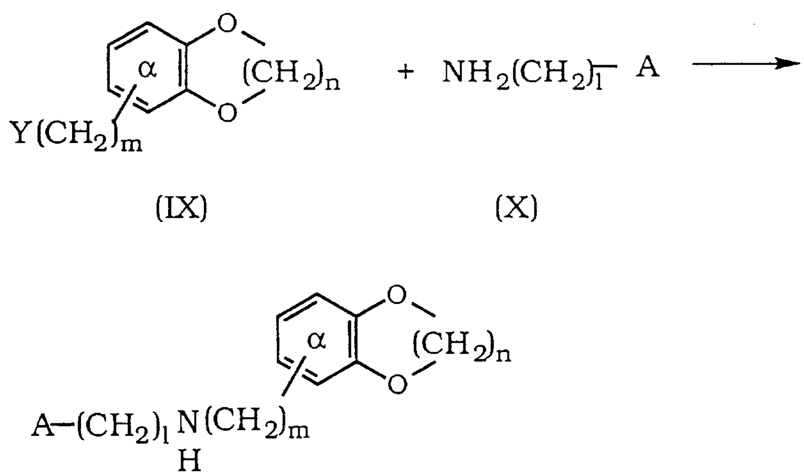

(IX) (X)

(XI)

(wherein, A, $\alpha$, 1, m and n are as defined m the above general formula (I); Y is as defined in relation to the above compound (III) or (IV)).

This process comprises condensation of the compound (IX) having a suitable leaving group with straight-chain alkyl amine (X) having an imidazole ring or a benzimidazole ring at the end which may have substituent to obtain the objective compound (XI).

This reaction may be carried out in water or organic solvent, or as neat generally in the presence of a base.

The bases used in this step include, for example, sodium hydroxide, potassium hydroxide, sodium carbonate, potassium carbonate, sodium hydride, sodium alkoxide, triethylamine, etc.

The usable organic solvents include, for example, acetonitrile, tetrahydrfuran, ether, acetone, toluene, benzene, dimethyl sulfoxide, dioxane, N,N-dimethylformamide, methanol, ethanol, isopropanol, tert-butanol, etc.

When the reaction is carried out using a neat material, good results may be obtained by using phase transfer catalyst. Such catalysts include, for example, aliquot 336, tetra-n-butylammonium bromide, tetra-n-octylammonium bromide, etc.

The reaction temperature and time may not be particularly limited. The reaction may be carried out at suitable temperature from ice-cooling to refluxing temperature for 15 minutes to 72 hours.

In this reaction, m is preferably 1.

Thus obtained compound may be purified by the conventional means such as extraction with solvent, separation by chromatography, crystallization, etc.

Since the N-substituted imidazole derivatives of the present invention have the pharmacological activity described below, they are expected to be as novel circulatory drugs having excellent anti-hypertensive, hypolipidaemic and hypoglycemic activity.

The compound of the present invention may be formulated into a pharmaceutical preparation suitable for administration by any route with a conventional carrier. For oral administration, the formulations may be presented as tablets, capsules, granules, powders or liquid preparations. The solid preparations for oral administration may be formulated using the conventional excipients, binding agents, lubricants, as well as coloring agents, disintegrants. Excipients include, for example, lactose, starch, talc, magnesium stearate, crystalline cellulose, methyhlcellulose, carboxymethyl cellulose, glycerol, sodium alginate, gum arabic, etc. Binding agents include, for example, polyvinyl alcohol, polyvinyl ether, ethylcellulose, gum arabic, shellac, sucrose, etc; lubricants include, for example, magnesium stearate, talc, etc. In addition, conventional coloring agents or disintegrants may be used. The tablets may be coated according to the well known method. Liquid preparations may be aqueous or oily suspensions, solutions, syrups, elixirs, etc. and may be prepared according to the conventional method. For injection, the compound of the invention may be

compounded with pH regulators, buffers, stabilizer, isotonic agents, local anesthetics, etc. to prepare subcutaneous, intramuscular, intravenous injectable solution according to the conventional method. Oily bases such as cocoa butter, polyethylene glycol, lanolin, aliphatic acid triglyceride, witepsol (Trade name of Dynamite Nobel), etc. may be used to prepare suppository.

The dosage of thus obtained preparation may vary depending on the condition, weight, age or the like of the patients, and can not be uniformly administered. Generally, however, the dosage as employed for adult human treatment will preferably range from about 10 to 2,000 mg per day. Such dosage is preferably divided into 1 to 4 times a day.

## Example

The preparation of N-substituted imidazole derivatives of the present invention will be further illustrated in detail in the following examples. But the examples do not limit the invention in any way.

### Synthesis 1

#### Preparation of 4-(3-chloropropoxy)-1,3-benzodioxole

Metallic sodium (460 mg) was added and dissolved in anhydrous ethanol (20 ml), to which was added 4-hydroxy-1,3-benzodioxole (2.5 g). The mixture was stirred at room temperature for 10 minutes. 1-Bromo-3-chloropropane (3.56 g) was added and heated under reflux for 12 hours. Alter cooling, the separated salt was removed by filtration, then the solvent was distilled off and the residue was purified by silica gel column chromatography eluting with n-hexane/ethyl acetate (19:1 by volume) to obtain the objective 4-(3-chloropropoxy)-1,3-benzodioxole (3.35 g, 86 %) as an oil.

NMR δ ($CDCl_3$): 2.22 (2H, tt), 3.73 (2H, t), 4.22 (2H, t), 5.93 (2H, s), 6.52 (2H, d), 6.75 (1H, t)

### Example 1

#### Preparation of 4-[3-(1-imidazolyl)propoxy]-1,3-benzodioxole (compound 1)

Sodium hydride (60 % oil, 132 mg) was added to a solution of imidazole (224 mg) in N,N-dimethylformamide (10 ml), and stirred at 110 °C for an hour. Subsequently, 4-(3-chloropropoxy)-1,3-benzodioxole (738 mg) prepared in Synthesis 1 was added and stirred at 110 °C for 5 hours. After the solvent was distilled off, the residue was separated by silica gel column chromatography eluting with methanol/chloroform (1:49 by volume), and the objective 4-[3-(1-imidazolyl)propoxy]-1,3-benzodioxole (compound 1) (700 mg, 95 %) was crystallized from ether.

m.p.: 63 - 65 °C

NMR δ ($CDCl_3$): 2.15 (2H, tt), 3.96 (2H, t), 4.13 (2H, t), 5.89 (2H, s), 6.42 (1H, d), 6.49 (1H, d), 6.70 (1H, dd), 6.88 (1H, s), 7.00 (1H, s), 7.44 (1H, s)

### Example 2

#### Preparation of 6-chloro-4-[3-(1-imidazolyl)propoxy]-1,3-benzodioxole (compound 2)

The compound obtained in Example 1 (1.0 g) was dissolved in DMF, to which was added sulfuryl chloride (0.39 ml) under ice cooling in a nitrogen flow. The temperature was raised to 110 °C and the mixture was stirred for 3 hours. After the solvent was distilled off, the residue was separated by silica gel column chromatography eluting with methanol/chloroform (1:49 by volume) to obtain the objective 6-chloro-4-[3-(1-imidazolyl)propoxy]-1,3-benzodioxole (compound 2) (280 mg, 25 %) as an oil.

NMR δ ($CDCl_3$): 2.04 (2H, tt), 4.04 (2H, t), 4.15 (2H, t), 5.83 (2H, s), 6.38 (1H, d), 6.72 (1H, d), 6.87 (1H, s), 6.94 (1H, s), 7.47 (1H, s)

### Example 3

#### Preparation of 4-[3-(2-methyl-1-imidazolyl)propoxy]-1,3-benzodioxole (compound 3)

The objective 4-[3-(2-methyl-1-imidazolyl)propoxy]-1,3-benzodioxole (compound 3)(400 mg, 66 %) was obtained as an oil from 2-methylimidazole (288 mg) and 4-(3-chloropropoxy)-1,3-benzodioxole (500 mg)

synthesized in Synthesis 1 by a procedure similar to that described in Example 1.

NMR δ ($CDCl_3$): 2.07 (2H, tt), 2.28 (3H, s), 3.93 (2H, t), 3.99 (2H, t), 5.85 (2H, s), 6.39 (1H, dd), 6.45 (1H, dd), 6.67 (1H, dd), 6.75 (1H, d), 6.81 (1H, d)

Example 4

Preparation of 4-[3-(4,5-dicyano-1-imidazolyl)propoxy]-1,3-benzodioxole (compound 4)

The objective 4-[3-(4,5-dicyano-1-imidazolyl)propoxy]-1,3-benzodioxole (compound 4)(600 mg, 87 %) was obtained from 4,5-dicyanoimidazole (325 mg) and 4-(3-chloropropoxy)-1,3-benzodioxole (500 mg) synthesized in Synthesis 1 by a procedure similar to that described in Example 1 (crystallized from ether).
m.p.: 178 - 181 °C

NMR δ (DMSO-$d_6$): 2.21 (2H, tt), 4.07 (2H, t), 4.34 (2H, t), 5.91 (2H, s), 6.54 (2H, d), 6.72 (1H, t), 8.32 (1H, s)

Example 5

Preparation of 4-[3-(4,5-dichloro-1-imidazolyl)propoxy]-1,3-benzodioxole (compound 5)

The objective 4-[3-(4,5-dichloro-1-imidazolyl)propoxy]-1,3-benzodioxole (compound 5) (460 mg, 63 %) was obtained from 4,5-dichloroimidazole (351 mg) and 4-(3-chloropropoxy)-1,3-benzodioxole (500 mg) synthesized in Synthesis 1 by a procedure similar to that described in Example 1 (crystallized from ether).
m.p.: 89 - 90 °C

NMR δ ($CDCl_3$): 2.12 (2H, tt), 3.96 (2H, t), 4.09 (2H, t), 5.87 (2H, s), 6.41 (2H, dd), 6.46 (1H, dd), 6.68 (1H, dd), 7.33 (1H, s)

Example 6

Preparation of 4-[3-(1-benzimidazolyl)propoxy]-1,3-benzodioxole (compound 6)

The objective 4-[3-(1-benzimidazolyl)propoxy]-1,3-benzodioxole (compound 6)(530 mg, 77 %) was obtained from benzimidazole (302 mg) and 4-(3-chloropropoxy)-1,3-benzodioxole (500 mg) synthesized in Synthesis 1 by a procedure similar to that described in Example 1 (crystallized from ether).
m.p.: 59 - 61 °C

NMR δ ($CDCl_3$): 2.23 (2H, tt), 3.93 (2H, t), 4.34 (2H, t), 5.89 (2H, s), 6.40 (1H, d), 6.50 (1H, d), 6.70 (1H, dd), 7.24 (2H, m), 7.36 (1H, m), 7.77 (1H, m), 7.86 (1H, s)

Synthesis 2

Preparation of 4-(4-chlorobutoxy)-1,3-benzodioxole

Metallic sodium (127 mg) was added and dissolved in anhydrous ethanol (10 ml), to which was added 4-hydroxy-1,3-benzodioxole (690 mg), and stirred at room temperature for 10 minutes. 4-Chlorobutyl p-toluenesulfonate (1.55 g) was added and heated under reflux for 12 hours. After work-up in the same manner as Synthesis 1, the objective 4-(4-chlorobutoxy)-1,3-benzodioxole (1.08 g, 95%) was obtained as an oil.

NMR δ ($CDCl_3$): 1.95 (4H, m), 3.60 (2H, t), 4.10 (2H, t), 5.93 (2H, s), 6.50 (1H, d), 6.51 (1H, d), 6.75 (1H, dd)

Example 7

Preparation of 4-[4-(1-imidazolyl)butoxy]-1,3-benzodioxole (compound 7)

The objective 4-[4-(1-imidazolyl)butoxy]-1,3-benzodioxole (compound 7)(490 mg, 86 %) was obtained as an oil from 4-(4-chlorobutoxy)-1,3-benzodioxole (500 mg) synthesized in Synthesis 2 by a procedure similar to that described in Example 1.

NMR δ ($CDCl_3$): 1.73 (2H, tt), 1.94 (2H, tt), 3.98 (2H, t), 4.04 (2H, t), 5.89 (2H, s), 6.43 (1H, dd), 6.48 (1H, dd), 6.71 (1H, dd), 6.89 (1H, d), 7.00 (1H, d), 7.45 (1H, s)

Synthesis 3

Preparation of 4-(5-chloropentoxy)-1,3-benzodioxol

By the reaction and work-up similar to those described in Synthesis 1 using 5-chloropentyl p-toluenesulfonate (1.64 g), the objective 4-(5-chloropentoxy)-1,3-benzodioxole (730 mg, 60 %) was obtained as an oil.

NMR δ ($CDCl_3$): 1.59 (2H, tt), 1.79 (4H, m), 3.51 (2H, t), 4.03 (2H, t), 5.89 (2H, s), 6.47 (2H, d), 6.71 (1H, t)

Example 8

Preparation of 4-[5-(1-imidazolyl)pentoxy]-1,3-benzodioxole (compound 8)

The objective 4-[5-(1-imidazolyl)pentoxy]-1,3-benzodioxole (compound 8)(700 mg, 85 %) was obtained from 4-(5-chloropentoxy)-1,3-benzodioxole (730 mg) synthesized in Synthesis 3 (crystallized from ether).
m.p.: 51 - 53 °C

NMR δ ($CDCl_3$): 1.45 (2H, tt), 1.80 (4H, m), 3.91 (2H, t), 4.02 (2H, t), 5.90 (2H, s), 6.45 (1H, d), 6.48 (1H, d), 6.72 (1H, t), 6.87 (1H, s), 7.01 (1H, s), 7.43 (1H, s)

Synthesis 4

Preparation of 5-(3-chloropropoxy)-1,3-benzodioxole

By the reaction and work-up similar to those described in Synthesis 1 using sesamol (2.07 g), the objective 5-(3-chloropropoxy)-1,3-benzodioxole (2.00 g, 62 %) was obtained as an oil.

NMR δ ($CDCl_3$): 2.18 (2H, tt), 3.71 (2H, t), 4.02 (2H, t), 5.90 (2H, s), 6.31 (1H, dd), 6.47 (1H, d), 6.68 (1H, d)

Example 9

Preparation of 5-[3-(1-imidazolyl)propoxy]-1,3-benzodioxole (compound 9)

The objective 5-[3-(1-imidazolyl)propoxy]-1,3-benzodioxole (compound 9)(2.04 g 89 %) was obtained from 5-(3-chloropropoxy)-1,3-benzodioxole (2.00 g) synthesized in Synthesis 4 by a procedure similar to that described in Example 1 (crystallized from ether).
m.p.: 56 - 58 °C

NMR δ ($CDCl_3$): 2.14 (2H, tt), 3.78 (2H, t), 4.13 (2H, t), 5.88 (2H, s), 6.24 (1H, dd), 6.43 (1H, d), 6.66 (1H, d), 6.87 (1H, d), 7.02 (1H, d), 7.43 (1H, s)

Synthesis 5

Preparation of 4-(3-chloropropoxymethyl)-1,3-benzodioxole

1-Bromo-3-chloropropane (647 mg) was added to a solution of 4-hydroxymethyl-1,3-benzodioxole (500 mg) and sodium hydride (60 % oil, 145 mg) in DMF, and the mixture was stirred at 110 °C for 6 hours.

The solvent was distilled off, and the residue was separated by silica gel column chromatography eluting with ethyl acetate/n-hexane (1:9 by volume) to obtain the objective 4-(3-chloropropoxymethyl)-1,3-benzodioxole (220 mg, 29 %) as an oil.

NMR δ ($CDCl_3$): 2.03 (2H, tt), 3.61 (2H, t), 3.64 (2H, t), 4.50 (2H, s), 5.96 (2H, s), 6.80 (3H, m)

Example 10

Preparation of 4-[3-(1-imidazolyl)propoxymethyl]-1,3-benzodioxole (compound 10)

The objective 4-[3-(1-imidazolyl)propoxymethyl]-1,3-benzodioxole (compound 10)(180 mg, 72 %) was obtained as an oil from 4-(3-chloropropoxymethyl)-1,3-benzodioxole (220 mg) synthesized in Synthesis 5 by a procedure similar to that described in Example 1.

NMR δ ($CDCl_3$): 2.00 (2H, tt), 3.40 (2H, t), 4.05 (2H, t), 4.56 (2H, s), 5.95 (2H, s), 6.81 (3H, m), 6.88 (1H, s), 7.02 (1H, d), 7.44 (1H, s)

Example 11

Preparation of N-[3-(1-imidazolyl)propyl]-2,3-methylenedioxybenzamide (compound 11)

Thionyl chloride (0.8 ml) and DMF (0.02 ml) were added to a solution of 2,3-methylenedioxybenzoic acid (1.22 g) in 1,2-dichloroethane (5 ml), and the mixture was heated under reflux in a nitrogen flow for an hour. The solvent was distilled off under reduced pressure. The residue was dissolved in methylene chloride (10 ml) and added dropwise to triethylamine (1.02 ml) and N-(3-aminopropyl)imidazole (0.63 g) in methylene chloride (10 ml) while cooling with ice, stirred in a nitrogen flow for an hour, then poured into water. The organic layer was washed with aqueous saturated sodium bicarbonate and brine, then dried over anhydrous magnesium sulfate. The solvent was distilled off and the residue was separated by silica gel column chromatography eluting with chloroform/methanol (19:1 by volume) to obtain the objective N-[3-(1-imidazolyl)propyl]-2,3-methylenedioxybenzamide (compound 11)(1.3 g, 65 %) as an oil.

NMR δ ($CDCl_3$): 2.06 (2H, tt), 3.41 (2H, dt), 3.99 (2H, t), 6.03 (2H, s), 6.91 (3H, m), 7.00 (2H, s), 7.47 (2H, m)

Example 12

Preparation of N-[2-(1-imidazolyl)ethyl]-3,4-methylenedioxybenzamide (compound 12)

The objective N-[2-(1-imidazolyl)ethyl]-3,4-methylenedioxybenzamide (compound 12) (1.36 g 45 %) was obtained from 3,4-methylenedioxybenzoic acid (1.94 g) and N-(2-aminoethyl)imidazole (1.30 g) by a procedure similar to that described in Example 11 (crystallized from ether).
m.p. 178 - 179 °C

NMR δ ($CDCl_3$): 3.70 (2H, dt), 4.20 (2H, t), 6.01 (2H, s), 6.40 (1H, t), 6.79 (1H, d), 6.91 (1H, s), 7.05 (1H, s), 7.22 (2H, m), 7.44 (1H, s)

Example 13

Preparation of N-[3-(1-imidazolyl)propyl]-3,4-methylenedioxybenzamide (compound 13)

The objective N-[3-(1-imidazolyl)propyl]-3,4-methylenedioxybenzamide (compound 13) (5.90 g, 86 %) was obtained from 3,4-methylenedioxybenzoic acid (4.15 g) and N-(3-aminopropyl)imidazole (3.13 g) by a procedure similar to that described in Example 11 (crystallized from ether).
m.p. 131 - 133 °C

NMR δ ($CDCl_3$): 2.10 (2H, tt), 3.44 (2H, dt), 4.04 (2H, t), 6.01 (2H, s), 6.15 (1H, t), 6.80 (1H, d), 6.96 (1H, s), 7.06 (1H, s), 7.23 (2H, m), 7.54 (1H, s)

Example 14

Preparation of N-[4-(1-imidazolyl)butyl]-2,3-methylenedioxybenzamide (compound 14)

The objective N-[4-(1-imidazolyl)butyl]-3,4-methylenedioxybenzamide (compound 14) (1.57 g, 68 %) was obtained from 3,4-methylenedioxybenzoic acid (1.33 g) and N-(4-aminobutyl)imidazole (1.11 g) by a procedure similar to that described in Example 11.
m.p. 84 - 85 °C

NMR δ ($CDCl_3$): 1.56 (2H, tt), 1.82 (2H, tt), 3.42 (2H, dt), 3.97 (2H, t), 6.00 (2H, s), 6.16 (1H, t), 6.79 (1H, d), 6.89 (1H, s), 7.03 (1H, s), 7.24 (2H, m), 7.44 (1H, s)

Example 15

Preparation of N-[3-(1-imidazolyl)propyl]-5-methoxy-3,4-methylenedioxybenzamide (compound 15)

The objective N-[3-(1-imidazolyl)propyl]-5-methoxy-3,4-methylenedioxybenzamide (compound 15) (770 mg, 70 %) was obtained from 5-methoxy-3,4-methylenedioxybenzoic acid (710 mg) and N-(3-aminopropyl)-

imidazole (0.45 g) by a procedure similar to that described in Example 11 (crystallized from ether).
m.p. 121 - 122 °C

NMR δ ($CDCl_3$): 2.09 (2H, tt), 3.42 (2H, dt), 3.89 (3H, s), 4.02 (2H, t), 6.00 (2H, s), 6.72 (1H, t), 6.88 (1H, s), 6.95 (1H, s), 7.03 (1H, s), 7.08 (1H, s), 7.46 (1H, s)

Example 16

Preparation of N-[3-(1-imidazolyl)propyl]-1,4-benzodioxane-6-carboxamide (compound 16)

The objective N-[3-(1-imidazolyl)propyll-1,4-benzodioxane-6-carboxamide (compound 16) (1.84 g, 93 %) was obtained from 1,4-benzodioxane-6-carboxylic acid (1.24 g) and N-(3-aminopropyl)imidazole (0.86 g) by a procedure similar to that described in Example 11 (crystallized from ether).
m.p. 117 - 119 °C

NMR δ ($CDCl_3$): 2.05 (2H, tt), 3.38 (2H, dt), 3.99 (2H, t), 4.23 (4H, m), 6.82 (1H, d), 6.92 (1H, s), 7.01 (2H, s), 7.26 (1H, dd), 7.33 (1H, d), 7.44 (1H, s)

Example 17

Preparation of N-[2-(1-imidazolyl)ethyl]-3,4-methylenedioxyphenylacetamide (compound 17)

The objective N-[2-(1-imidazolyl)ethyl]-3,4-methylenedioxyphenylacetamide(compound 17) (360 mg, 26 %) was obtained from 3,4-methylenedioxyphenylacetic acid (919 mg) and N-(2-aminoethyl)imidazole (566 mg) by a procedure similar to that described in Example 11 (crystallized from ether).
m.p. 63 - 66 °C

NMR δ ($CDCl_3$): 3.34 (2H, s), 3.40 (2H, dt), 3.95 (2H, t), 5.82 (2H, s), 6.56 (1H, dd), 6.62 (1H, d), 6.65 (1H, d), 6.70 (1H, s), 6.83 (1H, s), 7.11 (1H, s), 7.45 (1H, t)

Example 18

Preparation of 5-[3-(1-imidazolyl)propylaminomethyl]-1,3-benzodioxole (compound 18)

N-(3-Aminopropyl)imidazole (1.88 g), 5-(chloromethyl)-1,3-benzodioxole (3.4 g) and triethylamine (1.4 ml) in toluene (10 ml) was heated under reflux for an hour, then cooled and the solvent was distilled off under reduced pressure. The residue was partitioned between chloroform/aqueous saturated sodium bicarbonate, and the chloroform layer was washed with saturated brine, dried over magnesium sulfate and distilled.

The residue was separated by silica gel column chromatography eluting with methanol/chloroform (1:9 by volume) to obtain the objective 5-[3-(1-imidazolyl)propylaminomethyl]-1,3-benzodioxole (compound 18) (350 mg, 9 %) as an oil.

NMR δ ($CDCl_3$): 1.85 (2H, tt), 2.21 (2H, t), 3.19 (2H, s), 3.52 (1H, brs), 3.99 (2H, t), 5.68 (2H, s), 6.50 (2H, m), 6.64 (1H, s), 6.71 (1H, s), 6.77 (1H, s), 7.26 (1H, s)

Experiment 1

Antihypertensive activity

Spontaneously hypertensive rats (SHR) were anesthetized with ether and polyethylene catheter for measurement of blood pressure was inserted in ventral aortas via caudal artery on the day before these experiments, and placed in separate cages and their tails were fixed. Under aparalytic conditions, blood pressure of semi-restrained rats was measured using a pressure transducer (TP-400T, manufactured by Nippon Koden). Heart rate was determined from pulse wave measured by a cardiotachometer (AT0691G, manufactured by Nippon Koden). On the test day, the test compounds were administered to rats after blood pressure and heart rate were confirmed to become sufficiently stable.

The test compounds were suspended in Tween 80 (1.0 % solution, 10 ml/kg) and orally administered to SHRs. The results are shown in Table 1.

The test compounds lowered blood pressure of SHR depending on dose, while they had no effect on heart rate.

Table 1

| Blood pressure lowering in SHR | | |
|---|---|---|
| Compound | Dose mg/kg | Maximum fall in blood pressure mmHg |
| 1 | 10<br>30<br>100 | -28.9<br>-69.8<br>-86.8 |
| 4 | 10 | -20 |
| 5 | 10 | -20 |
| 6 | 10 | -20 |
| 7 | 10 | -60 |
| 8 | 10 | -25 |
| 13 | 10<br>30<br>100 | -27.3<br>-50.5<br>-75.2 |
| 16 | 30 | -34.3 |

<u>Experiment 2</u>

<u>Anti-hyperlipemic activity</u>

Six-week old golden syrian hamsters (4 to 5 animals/group) were used.

Untreated control group received normal solid feed (MF, Oriental Kobo Kogyo), while control and a drug-treated groups received feed containing 1 % cholesterol and 0.5 % cholic acid (manufactured by Oriental Kobo Kogyo) to induce hypolipemia.

The test compound was suspended in 1.0 % Tween 80. The solvent or the test compound was started to be orally administered (10 ml/kg, once a day, for 5 days) as soon as feed was changed to cholesterol-containing feed.

Alter final administration, blood was drawn from abdominal descendens main vein while anesthetized with nembutal. Serum was collected and total cholesterol (TC) in serum was determined according to enzymatic method using assay kit (Kyowa Medics).

Inhibition of increase in TC was calculated using the following equation:

$$\text{Inhibition of increase in TC} = 100 - \frac{\text{TC of test compound-treated group} - \text{TC of untreated group}}{\text{TC of control group} - \text{TC of untreated control group}} \times 100$$

The results are shown in Table 2. The test compounds inhibited increase in TC depending on dose.

Table 2

| Anti-hyperlipemic activity | | | | | |
|---|---|---|---|---|---|
| Compound | Dose mg/kg | Inhibition of TC increase % | Compound | Dose mg/kg | Inhibition of TC increase % |
| 1 | 30<br>100 | 80<br>100 | 13 | 30<br>100 | 26<br>60 |
| 7 | 30 | 38 | 16 | 100 | 79 |

Experiment 3

Activity to lower blood sugar

Ten-week old KK-$A^y$ mice (4 - 6 animals/group) were used.

The solvent (1 % Tween 80 solution) or a suspension of the test compound was orally administered once a day for 11 days (10 ml/kg).

Before administration and after the final administration, blood was drawn from caudal vein, and serum was collected and blood sugar level in serum was determined.

The results are shown in Table 3. There was no difference between blood sugar levels before and after administration for control group. The test compound provided statistically significant decrease in blood sugar level for KK-$A^y$ mice compared with the value before administration. The change in weight of test compound-treated group during oral administration was almost same as that of the control group.

Table 3

| Hypoglycaemic activity | | |
|---|---|---|
| | Blood sugar level | |
| | Before administration | After administration |
| Control<br>Compound 1, 30 mg/kg | 676.7 ± 35.3<br>676.4 ± 33.7 | 690.5 ± 57.9<br>532.2 ± 23.9* |

*: $P < 0.05$ vs value before administration

## Claims

**1.** An N-substituted imidazole derivative of the following formula (I):

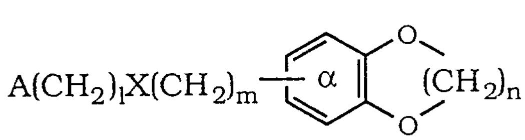

... (I)

wherein A is

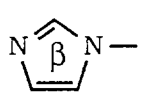 or 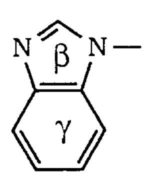 ;

X is -O-, -NHCO- or -NH-, 1 is an integer of 2 to 6; m is 0 or 1; n is 1 or 2; ring $\alpha$, ring $\beta$ and ring $\gamma$ are

independently unsubstituted or substituted by one or more substituents selected from the group consisting of a halogen atom, $C_1 \sim C_3$ alkyl, $C_1 \sim C_3$ alkoxy and cyano; or its pharmaceutically acceptable salt, or their hydrates.

**2.** The compound of Claim 1, wherein ring $\alpha$, ring $\beta$ and ring $\gamma$ are independently unsubstituted or substituted by one or more substituents selected from the group consisting from chlorine, methyl, methoxy and cyano.

**3.** The compound of Claim 1, wherein ring $\alpha$ is unsubstituted or substituted by chlorine or methoxy; ring $\beta$ is unsubstituted or substituted by chlorine, methyl or cyano; and ring $\gamma$ is unsubstituted.

**4.** The compound of Claim 1, wherein ring $\alpha$ is unsubstituted; ring $\beta$ is unsubstituted or substituted by chlorine or cyano; and ring $\gamma$ is unsubstituted.

**5.** The compound of Claim 1, wherein X is -O- or -NHCO-.

**6.** The compound of Claim 1, wherein X is -O-.

**7.** The compound of Claim 1, wherein 1 is an integer of 3 to 5.

**8.** The compound of Claim 1, wherein m is 0.

**9.** The compound of Claim 1, wherein n is 1.

**10.** The compound of Claim 1, wherein
A is

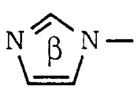

.

**11.** 4-[3-(4,5-dicyano-1-imidazolyl)propoxy]-1,3-benzodioxole or its salt.

**12.** 4-[3-(4,5-dichloro-1-imidazolyl)propoxy]-1,3-benzodioxole or its salt.

**13.** 4-[3-(1-benzimidazolyl)propoxy]-1,3-benzodioxole or its salt.

**14.** 4-[4-(1-imidazolyl)butoxy]-1,3-benzodioxole and its salt.

**15.** 4-[5-(1-imidazolyl)pentoxy]-1,3-benzodioxole and its salt.

**16.** N-[3-(1-imidazolyl)propyl]-1,4-benzodioxane-6-carboxamide and its salt.

**17.** A pharmaceutical composition which comprises as an active ingredient, an effective amount of the compound of Claim 1 together with pharmaceutically acceptable carriers therefor.

**18.** The pharmaceutical composition according to claim 17 for use in preventing or treating a disorder of hypertension.

**19.** The pharmaceutical composition according to claim 17 for use in preventing or treating a disorder of hyperglycemia.

**20.** The pharmaceutical composition according to claim 17 for use in preventing or treating a disorder of hyperlipidemia.

European Patent Office

# EUROPEAN SEARCH REPORT

Application Number
EP 94 11 7034

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 061 149 (MITSUBISHI CHEMICAL INDUSTRIES LTD.)<br>* claims *<br>--- | 1-20 | C07D405/12<br>A61K31/415 |
| A | EP-A-0 130 833 (YAMANOUCHI PHARMACEUTICAL CO. LTD.)<br>* claims *<br>--- | 1-20 | |
| A | EP-A-0 049 060 (BEECHAM GROUP LTD)<br>* claims *<br>----- | 1-20 | |

TECHNICAL FIELDS SEARCHED (Int.Cl.6)

C07D
A61K

The present search report has been drawn up for all claims

| Place of search | Date of completion of the search | Examiner |
|---|---|---|
| THE HAGUE | 6 January 1995 | Chouly, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)